# EUROPEAN PATENT APPLICATION

(11) **EP 3 797 756 A1**
(43) Date of publication of application: **31.03.2021**
(21) Application number: 20194286.9
(22) Date of filing: 03.09.2020
(51) Int. Cl.: A61K 8/02, A61K 8/73, A61Q 17/04

(54) **SUNSCREEN AGENT USING CELLULOSE NANOFIBERS**

(30) Priority: 25.09.2019 KR 20190118237
(71) Applicant: Nature Costech Co., Ltd., Chungcheongbuk-do 28578 (KR)
(72) Inventor: JEON, So Young, 28650 Chungcheongbuk-do (KR); IM, Da-Young, 28609 Chungcheongbuk-do (KR)
(74) Representative: Byrne, Declan

(57) **Abstract**

The present invention relates to a sunscreen agent using cellulose nanofibers, and more particularly, to a sunscreen agent composition containing cellulose nanofibers which are natural organic materials, thus to have blocking abilities against UV rays.

## Description

### [Technical Field]

The present invention relates to a sunscreen agent using cellulose nanofibers, and more particularly, to a sunscreen agent composition using cellulose nanofibers which are natural organic materials, thus to have blocking abilities against ultraviolet rays ('UV rays').

### [Background Art]

A skin of a human is sensitive to sunlight having wavelengths between about 290 nm and 400 nm. In particular, an ultraviolet B (UV-B) region having a wavelength between about 290 nm and 320 nm is known to cause skin damages accompanying redness, erythema, edema or the like. Prolonged or continuous exposure to the ultraviolet rays having the wavelength in the above-described range leads to severe skin diseases. In addition, an ultraviolet A (UV-A) region having a wavelength between about 320 nm and 400 nm is known to promote skin aging. Accordingly, recent sunscreen agents have been developed for obtaining a UV blocking effect through scattering, reflection, or absorption of the UV rays upon exposure to such UV rays.

Thereby, the sunscreen agents developed until recently have been studied for protection against UV-A and UV-B from a broad or narrow range of UV rays, retentive capacity relating to waterproofing and diaphoretic properties, ease of application, invisibility, non-contamination, ease of use such as slipping, and skin toxicity and skin stability caused by sunscreen ingredients and additives.

Effectiveness of the protection from sunlight is measured using a sun protection factor (SPF). Thereby, the sunscreen agents having SPF values developed until recently have increased public awareness of risks related to exposure to sunlight. Accordingly, a sunscreen agent having a high SPF value is required.

The current sunscreen agents require quantitative UV protection. Typically, zinc oxide and titanium dioxide are inorganic sunscreen materials that provide UV protection effects such as UV scattering and refraction, and oxymethylcinnamate, octocrylene, and the like are organic sunscreen materials that provide chemical UV protection effects.

However, the inorganic sunscreen agent is expensive, and when applied to the skin, white cloudiness occurs and feeling of use is poor. In addition, the inorganic sunscreen agent causes skin irritation due to active oxygen, and when used alone, it is difficult to obtain a high sunscreen index, and thereby requiring a large amount in use. In addition, in the case of the organic sunscreen agent, a product prepared by photoreaction causes skin inflammation, generates cancer cells, and leads to side effects such as DNA mutation and infertility, thereby causing safety problems in a human body.

Therefore, there is a need for a sunscreen agent having a high SPF and high protection abilities of skin from UV rays, while reducing concentrations of the inorganic ultraviolet ingredient and the organic ultraviolet ingredient in the above-described sunscreen agent.

### [Prior Art Document]

### [Patent Document]

(Patent Document 1) Korean Patent Laid-Open Publication No. 10-2009-0070454
(Patent Document 2) Korean Patent Laid-Open Publication No. 10-2017-0062126

### [Summary of Invention]

### [Problems to be Solved by Invention]

Accordingly, in consideration of the above-described circumstances, it is an object of the present invention to provide a sunscreen agent composition using cellulose nanofibers as a sunscreen boosting material, which have an effective SPF value while solving problems entailed in the existing organic sunscreen material and inorganic sunscreen material, at the time of using the organic sunscreen material or inorganic sunscreen material, which are sunscreen ingredients.

### [Means for Solving Problems]

According to an aspect of the present invention, there is provided a sunscreen agent composition including a sunscreen material and cellulose nanofibers having an average diameter of 5 to 50 nm and an average length of 100 nm to 2,000 nm.

Herein, the cellulose nanofiber may include any one of carboxymethyl cellulose nanofiber and TEMPO-oxidized cellulose nanofiber, or a combination thereof.

Further, the carboxymethyl cellulose nanofiber may have a degree of substitution from 0.01 to 1.5.

Further, the TEMPO-oxidized cellulose nanofiber may have a TEMPO oxidation amount from TEMPO 0.5 mM to TEMPO 1.5 mM.

Further, the sunscreen agent composition may include 0.01 to 25 % by weight of the sunscreen material and 0.01 to 25 % by weight of the cellulose nanofibers based on a total weight of the composition.

Furthermore, the sunscreen material may include: one or more inorganic sunscreen material selected from titanium dioxide, zinc oxide, iron oxide, silicon dioxide, magnesium oxide, manganese oxide, silica, alumina and aluminum oxide, or one or more organic sunscreen material selected from octyl-methoxycinnamate, butyl-methoxycinnamate, octinoxate and octocrylene.

### [Advantageous Effects]

According to the present invention as described above, the sunscreen agent composition using cellulose nanofibers may be usefully used as a sunscreen agent including a sunscreen material and a sunscreen boosting material, which have an effective SPF value while solving problems entailed in the existing organic sunscreen material and inorganic sunscreen material in the use.

The effects of the present invention as described above are not limited thereto, and other effects of the present invention will be clearly understood by persons having a common knowledge in the technical field to which the present invention pertains through examples and claims, which will be described below.

### [Brief Description of Drawings]

FIG. 1 is a graph illustrating results of an absorbance boosting test for ultraviolet A of carboxymethyl cellulose nanofibers of the present invention.
FIG. 2 is a graph illustrating results of an absorbance boosting test for ultraviolet B of the carboxymethyl cellulose nanofibers of the present invention.
FIG. 3 is a graph illustrating results of a transmittance boosting test for ultraviolet A of the carboxymethyl cellulose nanofibers of the present invention.
FIG. 4 is a graph illustrating results of a transmittance boosting test for ultraviolet B of the carboxymethyl cellulose nanofibers of the present invention.
FIG. 5 is a graph illustrating results of an absorbance boosting test for ultraviolet A of TEMPO-oxidized cellulose nanofibers of the present invention.
FIG. 6 is a graph illustrating results of an absorbance boosting test for ultraviolet B of the TEMPO-oxidized cellulose nanofibers of the present invention.
FIG. 7 is a graph illustrating results of a transmittance boosting test for ultraviolet A of the TEMPO-oxidized cellulose nanofibers of the present invention.
FIG. 8 is a graph illustrating results of a transmittance boosting test for ultraviolet B of TEMPO-oxidized cellulose nanofibers of the present invention.
FIG. 9 is a graph illustrating results of an absorbance boosting test for ultraviolet A of mixed cellulose nanofibers of the present invention.
FIG. 10 is a graph illustrating the results of an absorbance boosting test for ultraviolet B of the mixed cellulose nanofibers of the present invention.
FIG. 11 is a graph illustrating results of an absorbance boosting test for ultraviolet A of the carboxymethyl cellulose nanofibers of the present invention for each content.
FIG. 12 is a graph illustrating results of an absorbance boosting test for ultraviolet B of the carboxymethyl cellulose nanofibers of the present invention for each content.
FIG. 13 is a graph illustrating results of an absorbance boosting test for ultraviolet A of the TEMPO-oxidized cellulose nanofibers of the present invention for each content.
FIG. 14 is a graph illustrating results of an absorbance boosting test for ultraviolet B of the TEMPO-oxidized cellulose nanofibers of the present invention for each content.
FIG. 15 is a graph illustrating results of a stability test after applying a sunscreen agent containing the carboxymethyl cellulose nanofibers of the present invention.
FIG. 16 is a graph illustrating results of a spreadability test of the sunscreen agent containing the carboxymethyl cellulose nanofibers of the present invention.
FIG. 17 is a graph illustrating results of a stability test after applying a sunscreen agent containing the TEMPO-oxidized cellulose nanofibers of the present invention.
FIG. 18 is a graph illustrating results of a spreadability test of the sunscreen agent containing the TEMPO-oxidized cellulose nanofibers of the present invention.
FIG. 19 is a graph illustrating results of a smoothness test of the sunscreen agent containing the carboxymethyl cellulose nanofibers of the present invention.
FIG. 20 is a graph illustrating results of a smoothness test of the sunscreen agent containing the TEMPO-oxidized cellulose nanofibers of the present invention.

### [Mode for Carrying out Invention]

The present invention relates to a sunscreen agent using cellulose nanofibers, and more particularly, to a sunscreen agent composition using cellulose nanofibers which are natural organic materials, thus to have blocking abilities against UV rays. The present invention particularly discloses amplification and boosting of effective blocking abilities against UV rays.

The inventor of the present invention has found that, when using cellulose nanofibers as a sunscreen material and a sunscreen boosting material, which have an effective SPF value while solving problems entailed in the existing organic sunscreen material and inorganic sunscreen material, at the time of using the organic sunscreen material or the inorganic sunscreen material, which are sunscreen ingredients, UV blocking abilities and sunscreen boosting effect of the cellulose nanofibers could be obtained, and therefore, the present invention has been completed on the basis of the finding.

The 'composition' disclosed in the present invention may be produced in an effective cosmetic formulation having UV blocking abilities, and may be applied, for example, to sunscreen agents of various formulations such as lotions, creams, pastes, sprays, lip balms, sticks and the like.

In order for the sunscreen agent of the present invention to have an effective SPF value, in a hydrophilic surface which is treated with at least 5% by weight ('wt.%') or more of titanium dioxide, zinc oxide or a combination thereof, or is not treated, the sunscreen agent composition may be used in a concentration of at least 4 wt.% and suitably at least 25 wt.% to exceed at least SPF 15. The composition of the present invention exhibits efficient usability to a sunscreen ingredient, in particular, to zinc oxide or titanium dioxide. Preferably, fine-grained titanium dioxide or zinc oxide powders are variously used in a range of a few nm to a few hundred nm based on an average size of the particles, but it is not limited thereto.

As the sunscreen material disclosed in the present invention, an inorganic sunscreen material or an organic sunscreen material may be used. However, it is preferable to selectively use titanium dioxide and zinc oxide in terms of a size range of the particles, content and ratio of the sunscreen ingredients.

The sunscreen agent composition of the present invention may be used in a concentration of 0.01 to 25.0 wt% for the sunscreen material, and further includes cellulose nanofibers to have a synergistic effect of boosting the UV blocking abilities.

Herein, the cellulose nanofibers may achieve a high SPF value while resolving disadvantages such as whitening, skin spreadability, retentiveness, and skin toxicity of inorganic sunscreen ingredient and organic sunscreen ingredient, which are sunscreen materials. In particular, during using the cellulose nanofibers, application is allowed even at a very low content of the inorganic sunscreen ingredients or organic sunscreen ingredients. For example, even when containing the inorganic sunscreen ingredient in a very low concentration while not exceeding 25 wt.%, it is possible to achieve a sunscreen performance of effective SPF value by the cellulose nanofibers.

As an effective sunscreen ingredient in the present invention, any of organic sunscreen ingredient and inorganic sunscreen ingredient may be effectively used. For example, the organic sunscreen material may use benzophenone-s, homo salate, 4-methyl-benzylidene camphor, octylmethoxycinnamate (octinoxate), octyl-dimethyl-PABA, butyl methoxycinnamate, octocrylene, or a mixture thereof. In addition, the inorganic sunscreen material may use titanium dioxide, zinc oxide, iron oxide, silicon dioxide, magnesium oxide, manganese oxide, silica, alumina, aluminum oxide or a mixture thereof. Hereinafter, the present invention will be described as an example of using titanium dioxide, but it is not limited thereto.

According to the present invention, the cellulose nanofibers as a use of a sunscreen boosting material are disclosed to have an effective SPF value even when using a minimum amount of the sunscreen ingredient.

In the present invention, the cellulose nanofibers are fibers produced by cutting or degrading plant pulp to a nano size. The cellulose fiber of the present invention has a structure having an average diameter of 2 nm to 50 nm, and an average length of 100 to 2000 nm, and has an anionic functional group.

In the present invention, in relation to the size of the cellulose nanofibers, the fibers may have an average diameter of 2 nm or more and 200 nm or less, but preferably 2 nm or more and 150 nm or less, more preferably 2 nm or more and 100 nm or less, and particularly preferably 5 nm or more and 50 nm or less.

In the present invention, when the average diameter of the cellulose nanofibers is less than 2 nm, a viscosity of the cellulose nanofibers is increased, thereby causing a reduction in a dispersion stabilizing effect of an ultraviolet scattering agent. When the average diameter of the fibers exceeds 50 nm, a moisture content of cellulose may be reduced to cause a deterioration in rolling properties.

The cellulose nanofibers of the present invention have an average length of 50 nm to 2000 nm, but it is preferable to have an average length of 100 to 1000 nm in terms of capable of achieving a sufficient dispersion effect and a sunscreen boosting effect of the sunscreen ingredient.

The cellulose nanofibers may be produced by known methods, but the cellulose nanofibers of the present invention may be obtained by a specific method including the steps of suspending pulverized natural plant pulp or natural cellulose in water, and refining the same with a high pressure homogenizer, grinder or the like. The cellulose of the present invention is not limited to cellulose derived from cotton, plant, animal, or microorganism, but cellulose derived from plants such as coniferous or hardwood-derived kraft pulp or dissolved pulp, cotton linter, cellulose lignin, sawdust, vegetation cellulose, and the like may be used. Preferably, wood-based pulp such as coniferous-based pulp, hardwood-based pulp, cotton linter, etc., or non-wood-based pulp such as straw pulp and bagasse pulp may be used, and various other pulps may also be used.

The average diameter and length of the cellulose nanofibers may be measured in various ways. For example, a dispersion may be prepared by dispersing 0.05 to 0.1 wt.% in dry weight of cellulose in water, and the diameter and length of the dispersion may be measured by a transmission electron microscope (TEM) or a scanning electron microscope (SEM). At this time, the diameter and length thereof may be measured by using an electron microscope image with a suitable magnification. Specifically, one axis is defined as an arbitrary longitudinal or lateral width in the image, and the magnification is adjusted so that a plurality of fibers cross each other with respect to the axis. In addition, diameters and lengths of the fibers crossing a random axis in the image may be measured many times to calculate an average diameter and an average length of the fibers. In the present invention, it is necessary to identify nanofiber particles having somewhat uniform diameters and lengths so that the cellulose nanofibers have uniform dispersion forces and sunscreen boosting abilities, which may be achieved by appropriately controlling parameters in nanofiber processing.

The cellulose nanofibers in the present invention are an aqueous solution phase or an aqueous dispersion dispersed in water, and the concentration of cellulose nanofibers in the aqueous solution may be any concentration so long as it has sufficient dispersion. Usually, the concentration thereof is 5 wt.% or less in the aqueous solution, but the concentration of the cellulose nanofibers may be increased through a dehydration process.

It is preferable that the cellulose nanofibers have an anionic functional group. Specifically, examples of the cellulose having an anionic functional group may include cellulose oxide, carboxymethyl cellulose, polyvalent carboxymethyl cellulose, and long-chain carboxy cellulose. Preferably, cellulose oxide having a uniformity in hydroxyl group selectivity of the fiber surface, reaction conditions, and fiber diameter, or carboxymethyl cellulose or TEMPO-oxidized cellulose having high versatility and stability is used.

In the present invention, the cellulose nanofiber is prepared by mixing pulp which are a cellulose raw material with methanol, ethanol, n-propyl alcohol, isopropyl alcohol, n-butyl alcohol, isobutyl alcohol, t-butyl alcohol alone or in a combination of two or more thereof, and treating the mixture by adding sodium hydroxide or potassium hydroxide. The treated pulp is subjected to a treatment by mixing with acetic acid chloride and ethanol. At this time, the treatment is performed at a temperature of 50 to 90°C, preferably 75°C, for 10 minutes to 4 hours of a treatment time, and preferably 2 hours to 3 hours. Thereafter, the treated pulp is pulverized in a high pressure homogenizer and a grinder to produce cellulose nanofibers. Herein, the high pressure homogenizer or the grinder may use a commonly used high pressure homogenizer and a grinder, and the particles thereof may be ultra-refined in a size of nanometer (nm) depending on characteristics of the device.

The cellulose nanofibers produced in the present invention are prepared through carboxymethylation, and may be carboxymethyl cellulose nanofibers having a degree of substitution (DS) in a range of 0.01 to 1.5 that can be prepared, preferably, a degree of substitution in a range of 0.05 to 1.2, and more preferably, a degree of substitution in a range of 0.1 to 0.7. That is, when substituting the cellulose nanofibers with carboxy methyl groups, an electrical reaction force is generated between cellulose particles, and the cellulose nanofibers substituted with these carboxymethyl functional groups may be prepared in a size of nm having a sufficient dispersion force. Herein, if the degree of substitution of carboxymethyl is less than 0.1, it is difficult to decompose into cellulose nanofibers having a sufficient size, and thus transparency, dispersion stability and spreadability are reduced, whereas if the degree of substitution thereof exceeds 0.8, viscosity and spreadability are increased to cause a change in properties. Therefore, it is preferable to have a degree of substitution within the range of the present invention.

Further, in the present invention, the oxidized cellulose may use oxidized cellulose nanofibers which are oxidized using a compound such as TEMPO ((2,2,6,6-tetramethylpiperidin-1-yl)oxidanyl), wherein a preferred TEMPO oxidation amount is 0.5 to TEMPO 1.5 mM, and more preferably, the TEMPO oxidation amount is 0.7 to TEMPO 1.25 mM.

Preferably, the sunscreen material used in the present invention includes titanium dioxide or zinc oxide as an effective ingredient. In this case, when the sunscreen material has a particle size in a diameter of 10 nm to 200 nm or less, it is possible to obtain an effective sunscreen boosting effect of SPF value due to sufficient dispersion in mixing with the cellulose nanofibers together with an effective UV blocking effect.

Among the sunscreen agents of the present invention, it is preferable to contain titanium dioxide or zinc oxide, which is a sunscreen material, in an amount of 0.01 to 25 wt.%, and thereby, it is possible to amplify the UV blocking effect through dispersion stabilization of the sunscreen material due to sensitization of the cellulose nanofibers.

Specifically, in the sunscreen agent of the present invention, a content ratio of the sunscreen material and the cellulose nanofibers may be appropriately determined depending on stability and feeling of use of the sunscreen agent.

The sunscreen agent composition of the present invention may be uniformly mixed with the sunscreen material using cellulose nanofibers as a dispersion in the composition, and it is preferable to use a high pressure homogenizer. The sunscreen agent prepared as described above may be produced in a form of oil, oil in water, moisture, etc., and in various product forms such as lotions, creams, pastes, sprays, lip balms, sticks, etc., as a form of products.

Hereinafter, examples of the present invention will be described in more detail. However, the following examples are only intended to specifically illustrate the contents of the present invention, and the present invention is not limited thereto.

### <Example 1> Preparation of cellulose nanofibers

### 1-1 Preparation of carboxymethyl cellulose nanofibers

Carboxymethyl cellulose nanofibers were prepared through the present example. More specifically, anionization was induced in pulp functional groups by mixing 1500 ml of sodium hydroxide (NaOH) and ethanol in 100 g of pulp.

500 ml of monochloroacetic acid (MCA) and ethanol were mixed with the anionized pulp, then the mixture was subjected to a reaction at 75 °C for 2 hours. Herein, an addition amount of each of acetic acid chloride and sodium hydroxide for each degree of substitution (DS) 0.05 to 0.8 of carboxymethyl in cellulose nanofibers was used as shown in Table 1 below.

**[Table 1]**

| | DS 0.05 | DS 0.1 | DS 0.2 | DS 0.3 | DS 0.4 | DS 0.5 | DS 0.6 | DS 07 | DS 0.8 |
|---|---|---|---|---|---|---|---|---|---|
| Pulp (Unit: g) | 100 | 100 | 100 | 100 | 100 | 100 | 100 | 100 | 100 |
| MCA (Unit: g) | 3.61 | 7.22 | 14.44 | 21.66 | 28.88 | 36.11 | 43.33 | 50.55 | 57.77 |
| NaOH (Unit: g) | 1.23 | 2.46 | 4.93 | 7.40 | 9.87 | 12.34 | 14.81 | 17.28 | 19.75 |

After the reaction was completed, the pulp was sufficiently washed and treated twice in a grinder (Masuko Sangyo, Supermasscollider), and again passed through a high pressure homogenizer (GEA, Panda Plus) twice to prepare cellulose nanofibers. Then, an average particle size thereof was confirmed through a transmission electron microscope (ispxrf.co, SEM/XRF).

### 1-2 Preparation of TEMPO-oxidized cellulose nanofibers

TEMPO-oxidized cellulose nanofibers were prepared through the present example. More specifically, 100 g of pulp was mixed with 2.5 g of sodium bromide (NaBr), 0.5 g of TEMPO ((2,2,6,6-tetramethylpiperidin-1-yl)oxidanyl)) and 1500 ml of distilled water, followed by performing a reaction with sodium hypochlorite (NaClO) in 0.5 to 1.5 mmol for 1 hour to carboxylate by oxidizing cellulose functional groups. At this time, sodium hydroxide (NaOH) was added to the mixture as a pH buffer. Herein, an addition amount of sodium hypochlorite for each 0.5 to 1.5 mmol of TEMPO oxidation amount of cellulose nanofibers was used as shown in Table 2 below.

**[Table 2]**

| | TEMPO 0.5 mM | TEMPO 0.7 mM | TEMPO 1.25 mM | TEMPO 1.5 mM |
|---|---|---|---|---|
| Pulp (Unit: g) | 100 | 100 | 100 | 100 |
| NaClO (Unit: g) | 67.59 | 94.62 | 168.98 | 202.27 |

After the reaction was completed, the pulp was sufficiently washed and treated five times in a high pressure homogenizer (GEA, Panda Plus) to prepare TEMPO-oxidized cellulose nanofibers. Then, an average particle size thereof was confirmed through a transmission electron microscope (ispxrf.co, SEM/XRF).

### 1-3 Confirmation of properties of cellulose nanofibers

In order to confirm the properties of the cellulose nanofibers prepared in the present example, zeta-potential was measured by a zetasizer Nano Z (Malvern Panalytical Ltd.).

The measured zeta potential may be divided according to a potential difference exhibited by anionic functional groups in accordance with substitution or oxidation method in the prepared cellulose nanofibers. The zeta potential measured for each cellulosic nanofiber of the present example is shown in Table 3 below.

**[Table 3]**

| DS | DS 0.05 | DS 0.1 | DS 0.2 | DS 0.3 | DS 0.4 | DS 0.5 | DS 0.6 | DS 0.7 | DS 0.8 |
|---|---|---|---|---|---|---|---|---|---|
| Zeta-Potential (mV) | -18.3 | -25.7 | -29.2 | -33.7 | -33.7 | -41.2 | -45.4 | -49.7 | -54.2 |
| TEMPO | TEMPO 0.5 mM | TEMPO 0.7 mM | TEMPO 1.25 mM | TEMPO 1.5 mM | | | | | |
| Zeta-Potential (mV) | -29.6 | -36.7 | -42.7 | -49.2 | | | | | |

As shown in Table 3, it was confirmed that the zeta potential was increased according to the degree of substitution and a degree of oxidation of the cellulose nanofibers prepared in the present example. Thereafter, the cellulose nanofibers were used through the present example.

### <Example 2> Preparation of UV blocking dispersion using carboxymethyl cellulose nanofibers

The carboxymethyl cellulose nanofibers prepared in the present example were mixed with titanium dioxide (TiO₂), which is an inorganic sunscreen material, to prepare a 0.3% aqueous dispersion of carboxymethyl cellulose nanofibers. The titanium dioxide (TiO₂) used herein was SinaBT, Korea's product IndiMix SUN40T(O), and the zinc oxide (ZnO) used herein was SinaBT, Korea's product IndiMix SUN50Z(O).

Specifically, 100 g of an aqueous dispersion was prepared by mixing titanium dioxide and carboxymethyl cellulose nanofibers together with water (D.W) so that they contain 0.25 wt.% in dry weight and 0.3 wt.% in dry weight, respectively. Preparative examples of the respect dispersions are shown in Table 4 below.

**[Table 4]**

| | | Example 2-1 | Example 2-2 | Example 2-3 | Example 2-4 | Example 2-5 | Example 2-6 | Example 2-7 | Example 2-8 | Example 2-9 | Comparat ive Example 1 | Comparat ive Example 2 |
|---|---|---|---|---|---|---|---|---|---|---|---|---|
| Cellulose nanofiber | Degree of substitution (DS) | 0.05 | 0.1 | 0.2 | 0.3 | 0.4 | 0.5 | 0.6 | 0.7 | 0.8 | - | Methyl cellulose |
| | Content (wt.%) | 0.3 | 0.3 | 0.3 | 0.3 | 0.3 | 0.3 | 03 | 0.3 | 0.3 | | 0.3 |
| Sunscreen agent | Titanium dioxide (TiO₂) (wt.%) | 0.25 | 0.25 | 0.25 | 0.25 | 0.25 | 0.25 | 0.25 | 0.25 | 0.25 | 0.25 | 0.25 |
| Water (D.W) | | q.s | q.s | q.s | q.s | q.s | q.s | q.s | q.s | q.s | q.s | q.s |

Comparative Example 1 prepared a dispersion without adding cellulose nanofiber, and Comparative Example 2 used methylcellulose instead of the cellulose nanofiber. The methylcellulose used herein was a commercial product of JUNSEI Co.

### <Example 3> Preparation of UV blocking dispersion using TEMPO-oxidized cellulose nanofibers

The TEMPO-oxidized cellulose nanofibers prepared in the present example were mixed with titanium dioxide (TiO₂), which is an inorganic sunscreen material, to prepare 0.3% of aqueous dispersion of TEMPO-oxidized cellulose nanofibers. The titanium dioxide (TiO₂) used herein was SinaBT, Korea's product IndiMix SUN40T(O), and the zinc oxide (ZnO) used herein was SinaBT, Korea's product IndiMix SUN50Z(O).

Specifically, 100 g of aqueous dispersion was prepared by mixing titanium dioxide and TEMPO-oxidized cellulose nanofibers together with water (D.W) so that they contain 0.25 wt.% in dry weight and 0.3 wt.% in dry weight, respectively. Preparative examples of the respect dispersions are shown in Table 5 below.

**[Table 5]**

| | | Example 3-1 | Example 3-2 | Example 3-3 | Example 3-4 | Comparative Example 1 | Comparative Example 2 |
|---|---|---|---|---|---|---|---|
| Cellulose nanofiber | TEMPO oxidation amount | TEMPO 0.5 mM | TEMPO 0.7 mM | TEMPO 1.25 mM | TEMPO 1.5 mM | - | Methyl cellulose |
| | Content (wt.%) | 0.3 | 0.3 | 0.3 | 0.3 | - | 0.3 |
| Sunscreen agent | Titanium dioxide (TiO₂) (wt.%) | 0.25 | 0.25 | 0.25 | 0.25 | 0.25 | 0.25 |
| Water (D.W) | | q.s | q.s | q.s | q.s | q.s | q.s |

Comparative Example 1 prepared a dispersion without adding cellulose nanofiber, and Comparative Example 2 used methyl cellulose instead of the cellulose nanofiber. The methylcellulose used herein was a commercial product of JUNSEI Co.

### <Example 4> Measurement of UV absorbance and UV transmittance

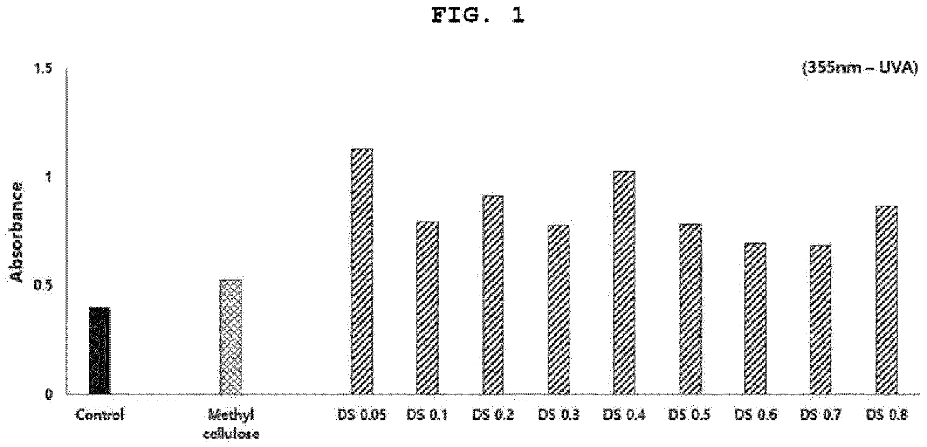

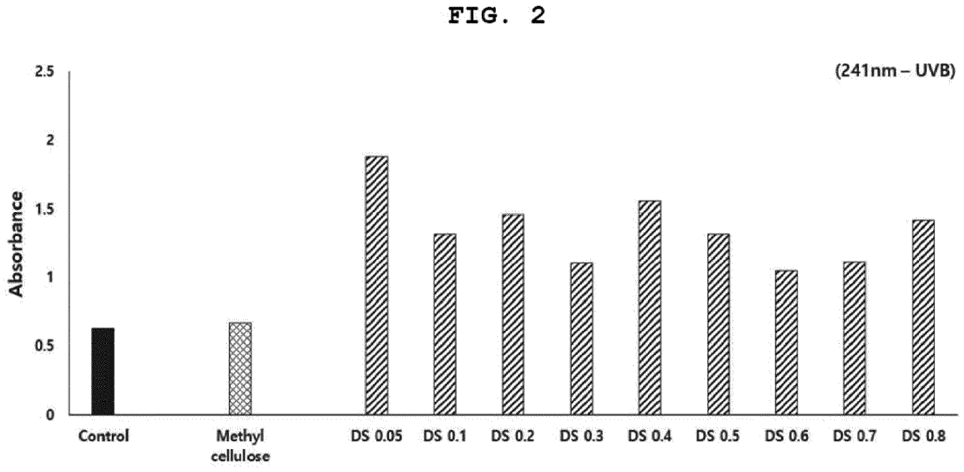

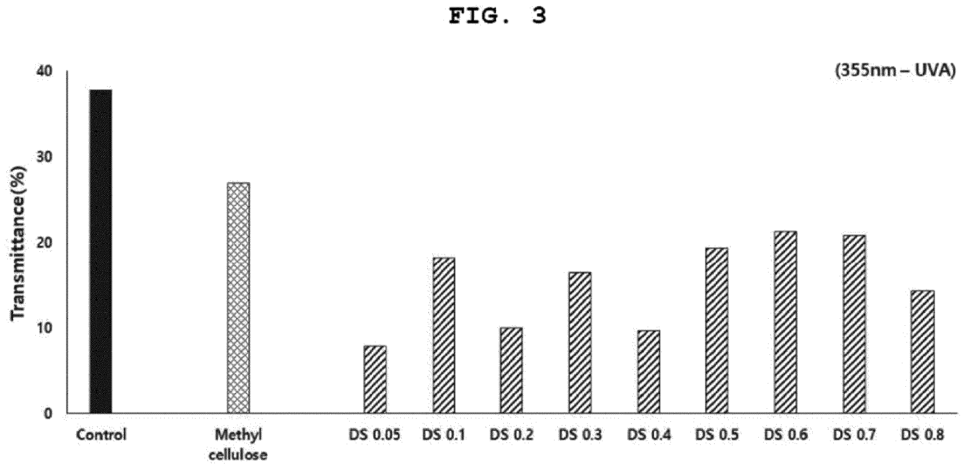

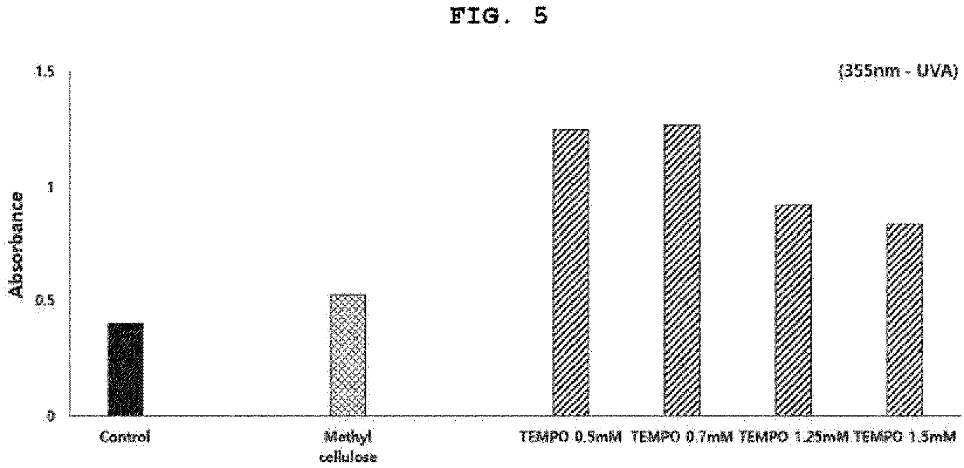

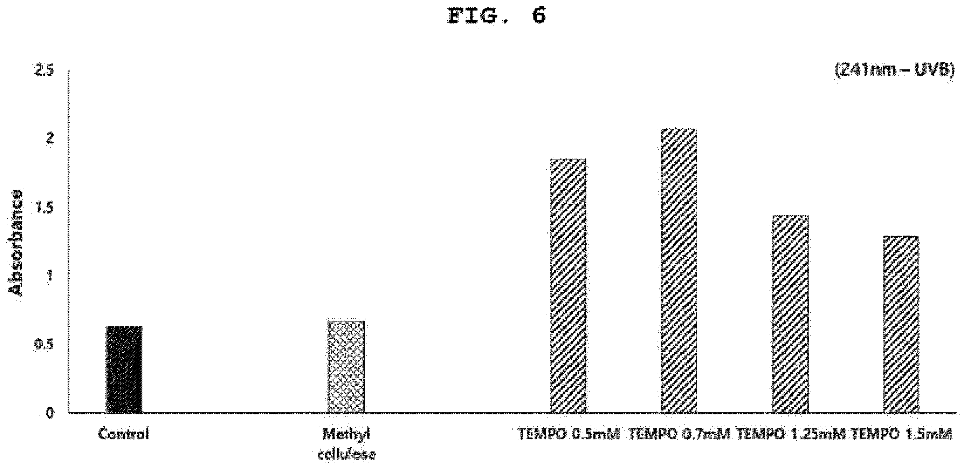

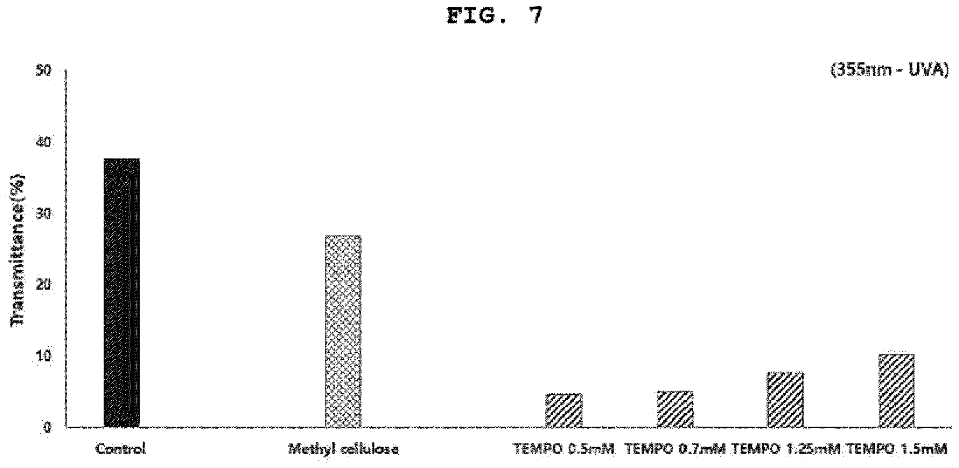

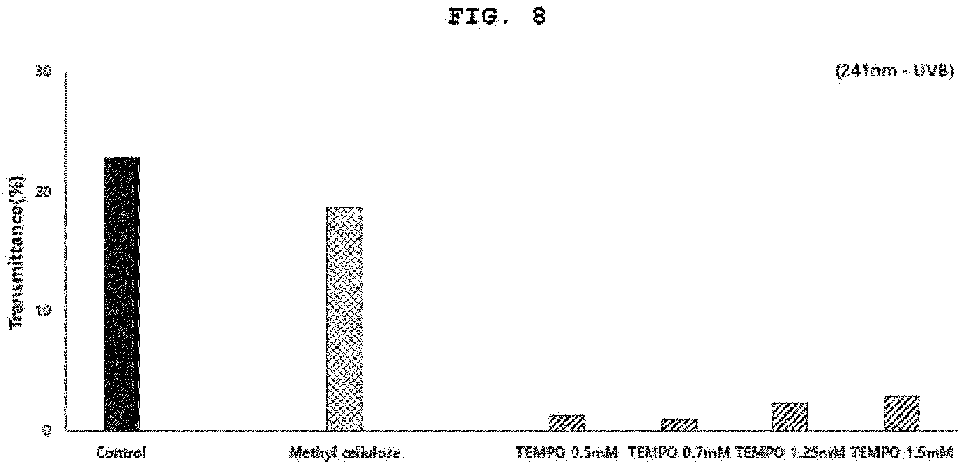

UV absorbance and UV transmittance of the dispersions prepared in Examples 2 and 3 were measured using a spectrophotometer (T60 UV/Visible Spectrophotometer of PG Instruments Co.). The wavelength during measuring was 335 nm and 241 nm in UV-A region and UV-B region, respectively, and the measured results are illustrated in FIGS. 1 to 8.

According to FIGS. 1 to 4, it was confirmed that the carboxymethyl cellulose nanofiber dispersion prepared in Example 2 had higher absorbance and lower transmittance than Comparative Examples 1 and 2, thereby exhibiting a boosting effect of the UV blocking abilities in the sunscreen material.

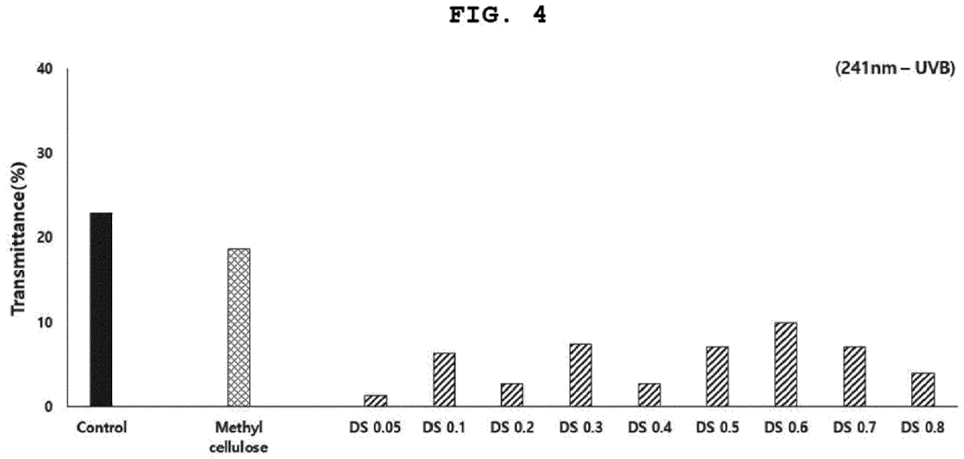

According to FIGS. 4 to 8, it was confirmed that the TEMPO-oxidized cellulose nanofiber dispersion prepared in Example 3 had higher absorbance and lower transmittance than Comparative Examples 1 and 2, thereby exhibiting a boosting effect of the UV blocking abilities in the sunscreen material.

### <Example 5> Preparation of UV blocking dispersion with carboxymethyl cellulose nanofibers and TEMPO-oxidized cellulose nanofibers mixed therein

The carboxymethyl cellulose nanofibers (DS 0.4) and TEMPO-oxidized cellulose nanofibers (TEMPO 0.7 mM) prepared in the present example were mixed, and the mixture was mixed with titanium dioxide (TiO₂), which is an inorganic sunscreen material, to prepare 0.3% aqueous dispersion of cellulose nanofibers. The titanium dioxide (TiO₂) used herein was SinaBT, Korea's product IndiMix SUN40T(O).

Specifically, 100 g of dispersion was prepared by mixing titanium dioxide and cellulose nanofibers together with water (D.W) so that they contain 0.25 wt.% in dry weight and 0.3 wt.% in dry weight, respectively. Preparative examples of the dispersion using the mixed cellulose nanofibers are shown in Table 6 below.

**[Table 6]**

| | | Example 5-1 | Example 5-2 | Example 5-3 | Example 5-4 | Example 5-5 | Comparative Example 1 | Comparative Example 2 |
|---|---|---|---|---|---|---|---|---|
| Cellulose nanofiber | Content of carboxymethyl cellulose nanofibers (DS 0.4) (wt.%) | 0.25 | 0.20 | 0.15 | 0.1 | 0.05 | - | - |
| | TEMPO-oxidized cellulose nanofibers (TEMPO 07mM) (wt.%) | 0.05 | 0.10 | 0.15 | 0.20 | 0.25 | - | Methyl cellulose |
| | Total content (wt.%) | 0.3 | 0.3 | 0.3 | 0.3 | 0.3 | - | 0.3 |
| Sunscreen agent | Titanium dioxide (TiO₂) (wt.%) | 0.25 | 0.25 | 0.25 | 0.25 | 0.25 | 0.25 | 0.25 |
| Water (D.W) | | q.s | q.s | q.s | q.s | q.s | q.s | q.s |

Comparative Example 1 prepared a dispersion without adding cellulose nanofiber, and Comparative Example 2 used methyl cellulose instead of the cellulose nanofiber. The methylcellulose used herein was a commercial product of JUNSEI Co.

### <Example 6> Measurement of UV absorbance

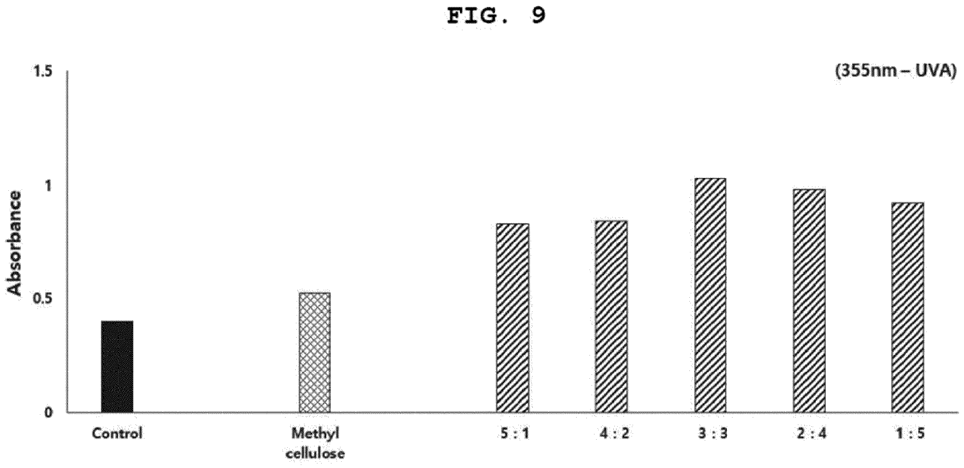

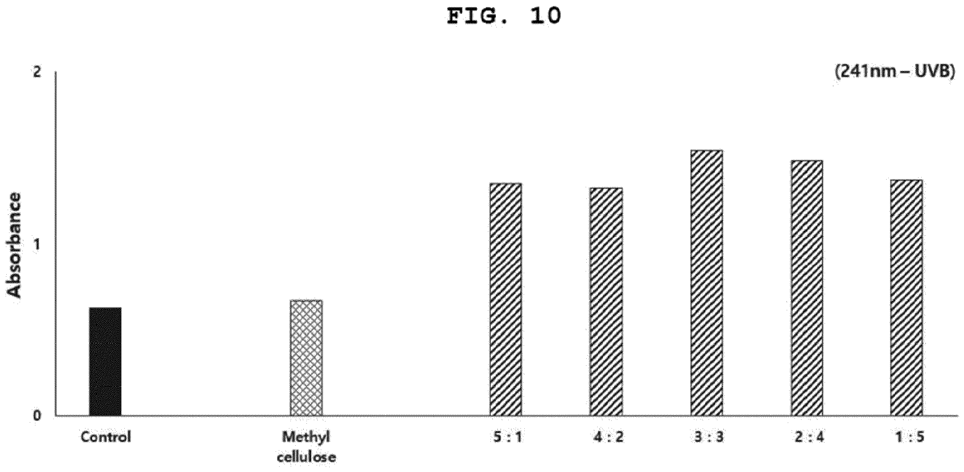

UV absorbance of the dispersion prepared in Example 5 was measured using a spectrophotometer (T60 UV/Visible Spectrophotometer of PG Instruments Co.). The wavelength during measuring was 335 nm and 241 nm in UV-A region and UV-B region, respectively, and the measured results are illustrated in FIGS. 9 and 10. As a result, it was confirmed that, even when using by mixing carboxymethyl cellulose nanofibers and TEMPO-oxidized cellulose nanofibers, the above dispersion had higher absorbance than Comparative Examples 1 and 2, thereby exhibiting a boosting effect of the UV blocking abilities in the sunscreen material.

### <Example 7> Measurement of absorbance for each content of cellulose nanofibers

Carboxymethyl cellulose nanofibers having 0.4 of a degree of substitution prepared in Example 1 and cellulose nanofibers having 0.7 mM of TEMPO oxidation amount were mixed with titanium dioxide (TiO₂), which is an inorganic sunscreen material, for each content to prepare 100 g of carboxymethyl cellulose nanofiber aqueous dispersion. Herein, preparative examples of the respective dispersions are shown in Table 7 below.

**[Table 7]**

| | | Example 7-1 | Example 7-2 | Example 7-3 | Example 7-4 | Example 7-5 | Example 7-6 | Example 7-7 | Example 7-8 | Comparati ve Example 1 | Comparati ve Example 2 |
|---|---|---|---|---|---|---|---|---|---|---|---|
| Cellulose nanofiber | Content of carboxymethyl cellulose nanofibers (DS 0.4) (wt.%) | 0.3 | 0.6 | 0.9 | 1.2 | - | - | - | - | - | Methyl cellulose |
| | TEMPO-oxidized cellulose nanofibers (TEMPO 0.7mM) (wt.%) | - | - | - | - | 0.3 | 0.6 | 0.9 | 1.2 | - | 0.3 |
| Sunscreen agent | Titanium dioxide (TiO₂) (wt.%) | 0.25 | 0.25 | 0.25 | 0.25 | 0.25 | 0.25 | 0.25 | 0.25 | 0.25 | 0.25 |
| Water (D.W) | | q.s | q.s | q.s | q.s | q.s | q.s | q.s | q.s | q.s | q.s |

Comparative Example 1 prepared a dispersion without adding cellulose nanofiber, and Comparative Example 2 used methyl cellulose instead of the cellulose nanofiber.

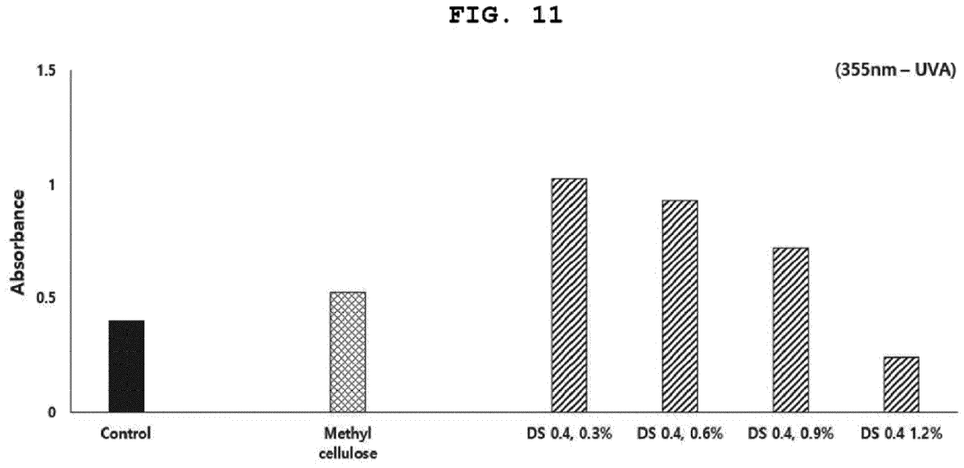

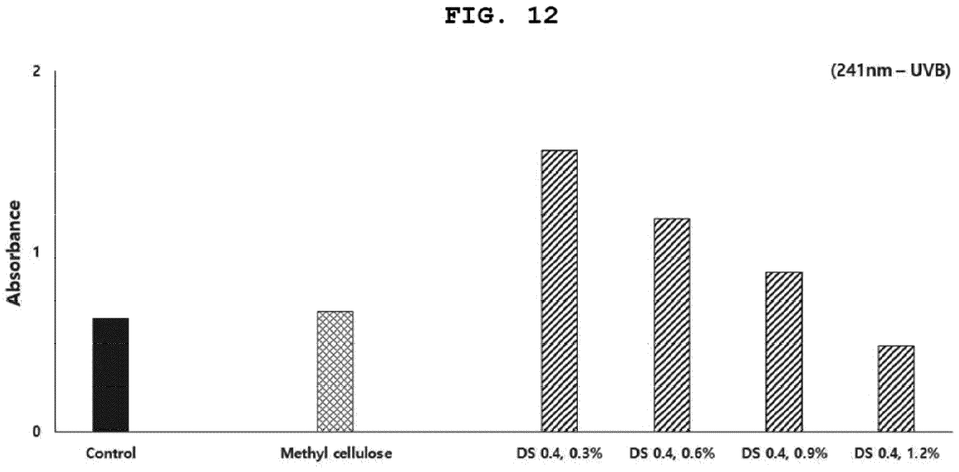

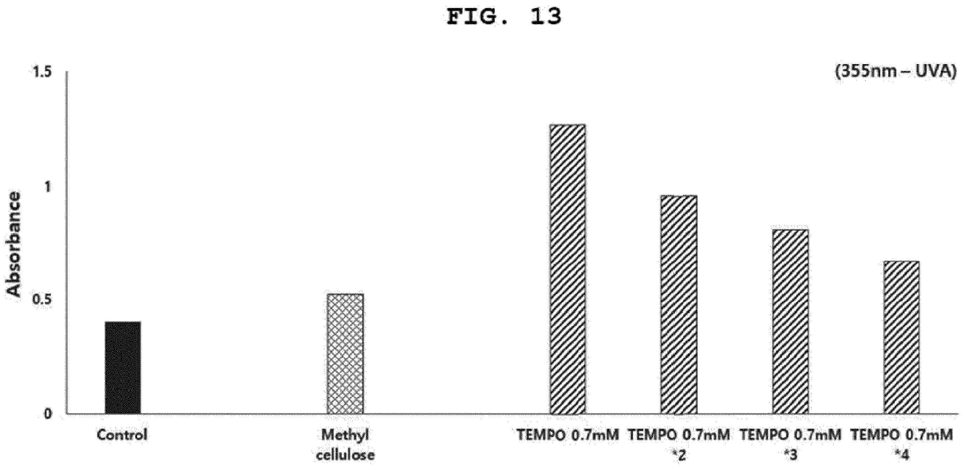

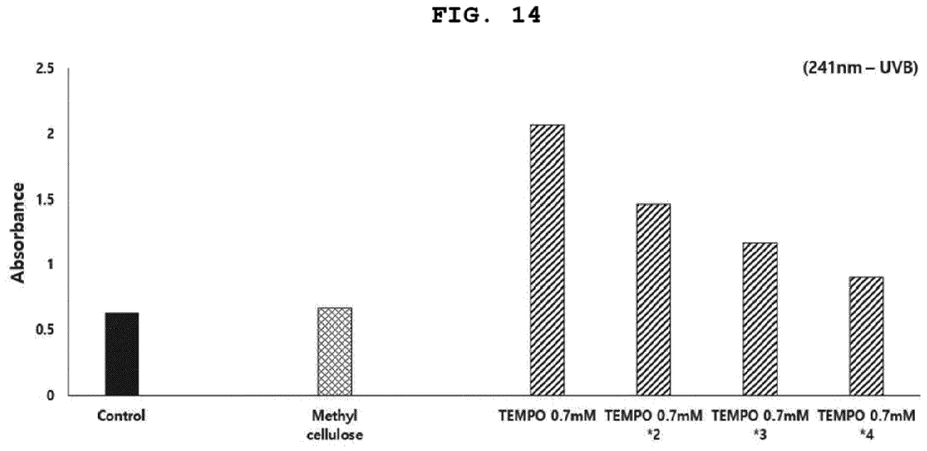

The absorbance of the respective dispersions were measured using a spectrophotometer (T60 UV/Visible Spectrophotometer of PG Instruments Co.). The wavelength during measuring was 335 nm and 241 nm in UV-A region and UV-B region, respectively, and the measured results are illustrated in FIGS. 11 to 14. As a result, it was confirmed that the prepared cellulose nanofibers had different absorbances for each content compared to Comparative Examples 1 and 2, and thereby exhibiting a boosting effect of the UV blocking abilities in the sunscreen material.

### <Example 8> Feeling of use of sunscreen agent formulation using cellulose nanofibers

Sunscreen agent were prepared using the respective cellulose nanofibers prepared in Example 1, and the spreadability and smoothness of the prepared sunscreen agent were measured.

More specifically, 100 g of sunscreen, which is a sunscreen agent, was prepared using carboxymethyl cellulose nanofibers, which are the cellulose nanofibers prepared in Example 1, and TEMPO-oxidized cellulose nanofibers. The contents and components prepared herein are shown in Table 8 below.

**[Table 8]**

| Component | Example 8-1 (Unit: g) | Example 8-2 (Unit: g) |
|---|---|---|
| Carboxymethyl cellulose nanofiber | 0.3 | - |
| TEMPO-oxidized cellulose nanofiber | - | 0.3 |
| Deionized water | q.s | q.s |
| Glycerine | 5 | 5 |
| 1,3 Butylene glycol | 3 | 3 |
| Cethyl ethyl hexanoate | 10 | 10 |
| Glyceryl stearate | 3.5 | 3.5 |
| Squalane | 2.5 | 2.5 |
| Dimethicone | 2 | 2 |
| Cetostearyl alcohol | 2.5 | 2.5 |
| Beeswax | 1 | 1 |
| Caprylyl glycol | 0.25 | 0.25 |
| 1,2 hexanediol | 0.25 | 0.25 |
| Titanium dioxide (IndiMix SUN40T(O)) | 2.5 | 2.5 |
| Zinc oxide (IndiMix SUN50Z(O)) | 2.5 | 2.5 |

Comparative Example 1 prepared a 100 g formulation without adding cellulose nanofiber, and Comparative Example 2 used methyl cellulose instead of the cellulose nanofiber.

By using the prepared sunscreen agent rheometer (Rheology), a storage modulus and a loss modulus of a static state representing spreadability and a dynamic state representing fixation property of the skin were measured. The rheometer used herein was Anton Paar's MCR 102 model, the static state was measured at shear stress of 1 Pa, and the static state was measured at shear stress of 120 Pa in a Liner viscoelastic range mode.

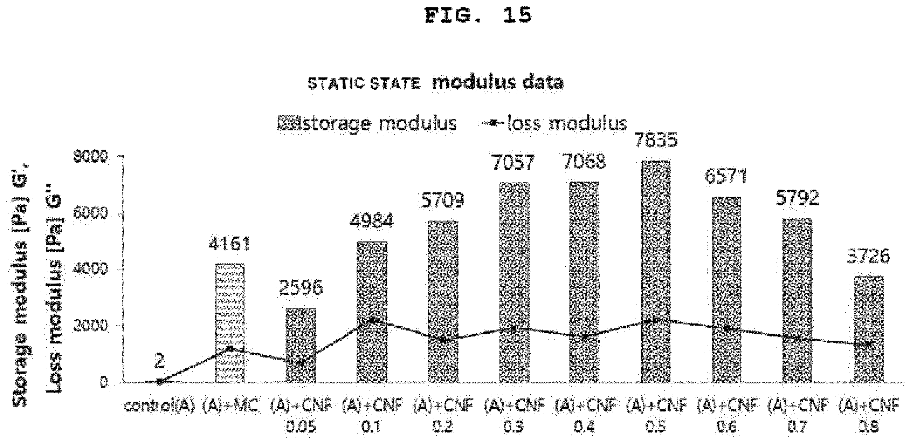

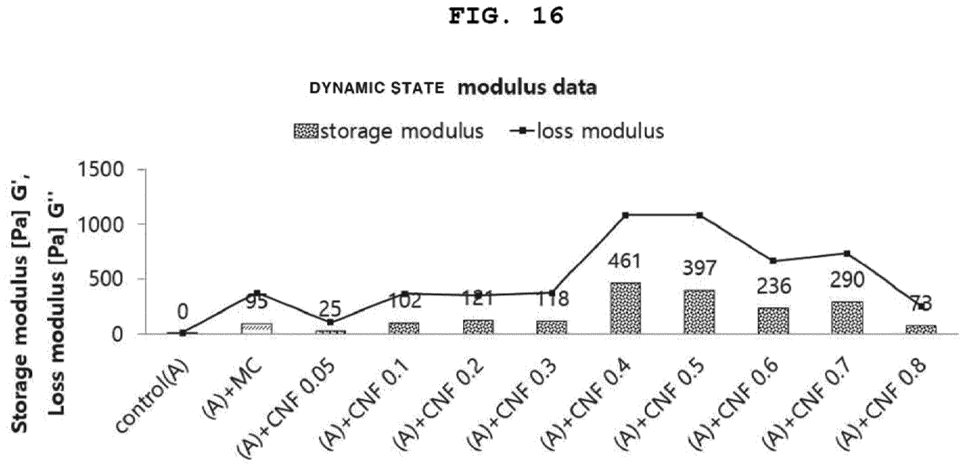

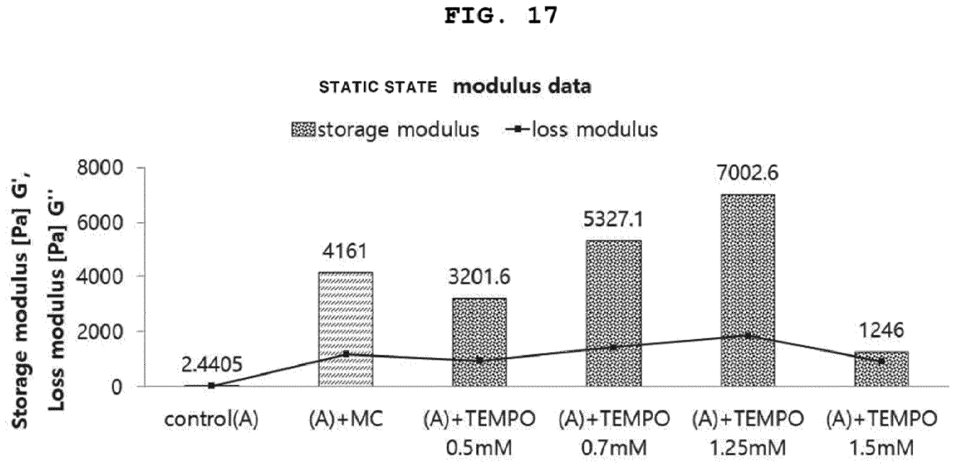

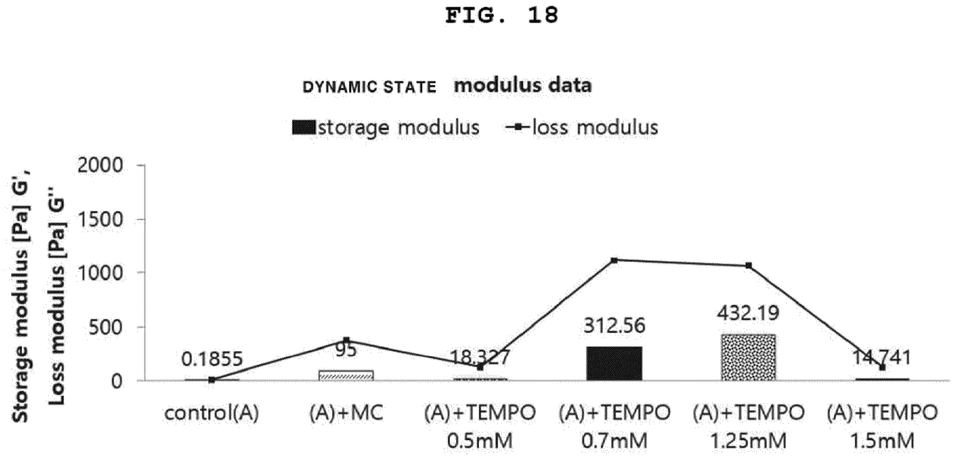

The rheometer measurement results of Example 8-1 are shown in FIGS. 15 and 16, and the rheometer measurement results of Example 8-2 are shown in FIGS. 17 and 18.

In the rheometer measurement results, G' value represents the storage modulus, G" value represents the loss modulus, and the storage modulus, wherein the storage modulus value represents elasticity, and the loss modulus value represents viscosity. If a difference value therebetween is small, it means that the sample has elastic properties, that is, similar to a solid, and if the difference value is large, it means that the sample has viscous properties, that is, similar to a liquid.

As a result, in the case of carboxymethyl cellulose nanofibers, it was confirmed that the formulation prepared with 0.1 to 0.7 of degree of substitution had good elasticity and viscosity compared to Comparative Example 1 and Comparative Example 2, and thereby exhibiting good spreadability.

In addition, in the case of TEMPO-oxidized cellulose nanofibers, it was confirmed that the formulation prepared with 0.7 and 1.2 mM of TEMPO oxidation amount had good elasticity and viscosity compared to Comparative Example 1 and Comparative Example 2, and thereby exhibiting good spreadability.

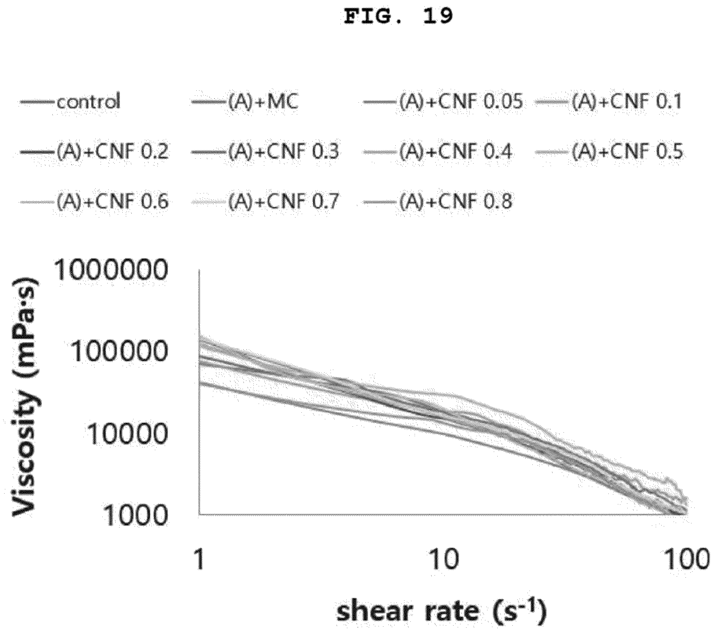

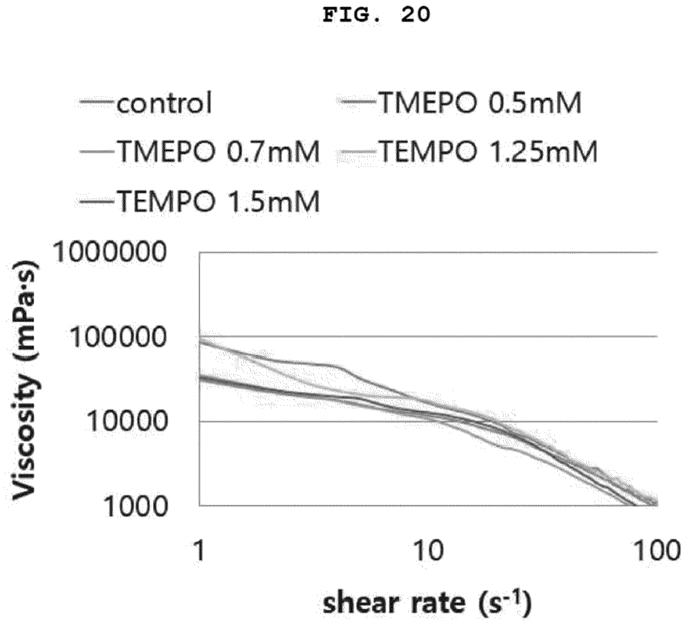

In order to confirm the smoothness of the formulations prepared in the present example, the rheometer was measured by using Anton Paar's MCR 102 model in a liner viscoelastic range mode, and the viscosity ranging from 1/s to 100/s was compared as a viscosity value for each shear rate. The rheometer measurement results are shown in Tables 9 and 10 below, and FIGS. 19 and 20.

**[Table 9]**

| Sample name | Control | (A)+MC | (A)+CNF 0.05 | (A)+CNF 0.1 | (A)+CNF 0.2 |
|---|---|---|---|---|---|
| Viscosity (mPa·S) | 86380 | 69858 | 42088 | 76782 | 145310 |
| (A)+CNF 0.3 | (A)+CNF 0.4 | (A)+CNF 0.5 | (A)+CNF 0.6 | (A)+CNF 0.7 | (A)+CNF 0.8 |
| 138060 | 124450 | 117070 | 155530 | 144200 | 40867 |

**[Table 10]**

| Sample name | Control | TEMPO 0.5 mM | TEMPO 0.7 mM | TEMPO 1.25 mM | TEMPO 1.5 mM |
|---|---|---|---|---|---|
| Viscosity (mPa·S) | 86380 | 31238 | 35401 | 95432 | 33224 |

As a result, in the case of carboxymethyl cellulose nanofibers, it was confirmed that, if the degree of substitution is less than 0.05 and the degree of substitution exceeds 0.8, the smoothness during applying was deteriorated compared to Comparative Example 1 and Comparative Example 2. In addition, in the case of TEMPO-oxidized cellulose nanofibers, it was confirmed that, if the degree of substitution is less than 0.7 and the degree of substitution exceeds 1.5, the smoothness during applying was deteriorated.

## Claims

1. A sunscreen agent composition comprising a sunscreen material and cellulose nanofibers having an average diameter of 5 to 50 nm and an average length of 100 nm to 2,000 nm.

2. The sunscreen agent composition according to claim 1, wherein the cellulose nanofiber comprises any one of carboxymethyl cellulose nanofiber and TEMPO-oxidized cellulose nanofiber, or a combination thereof.

3. The sunscreen agent composition according to claim 2, wherein the carboxymethyl cellulose nanofiber has a degree of substitution from 0.01 to 1.5.

4. The sunscreen agent composition according to claim 2, wherein the TEMPO-oxidized cellulose nanofiber has a TEMPO oxidation amount from TEMPO 0.5 mM to TEMPO 1.5 mM.

5. The sunscreen agent composition according to claim 1, comprising 0.01 to 25 % by weight of the sunscreen material and 0.01 to 25 % by weight of the cellulose nanofibers based on a total weight of the composition.

6. The sunscreen agent composition according to claim 1, wherein the sunscreen material comprises: one or more inorganic sunscreen material selected from titanium dioxide, zinc oxide, iron oxide, silicon dioxide, magnesium oxide, manganese oxide, silica, alumina and aluminum oxide; or one or more organic sunscreen material selected from octyl-methoxycinnamate, butyl-methoxycinnamate, octinoxate and octocrylene.
